# EUROPEAN PATENT SPECIFICATION

(11) **EP 0 998 279 B1**
(45) Date of publication and mention of the grant of the patent: **16.10.2002**
(21) Application number: 97921986.2
(22) Date of filing: 30.05.1997
(51) Int. Cl.: A61K 31/335, A61K 9/08, A61K 47/18, A61P 35/00

(54) **PHARMACEUTICAL INJECTION SOLUTION CONTAINING TAXOL**
TAXOL ENTHALTENDE PHARMAZEUTISCHE INJEKTIONSLÖSUNG
SOLUTION PHARMACEUTIQUE INJECTABLE CONTENANT DU TAXOL

(43) Date of publication of application: 10.05.2000
(73) Proprietor: Han, Man Woo, Suh-ku, Taejeon City (KR); Hong, Nam Doo, Mapo-ku, Seoul (KR); Yoo, Jae Kuk, Suh-ku, Taejeon City (KR)
(72) Inventor: Han, Man Woo, Suh-ku, Taejeon City (KR); Hong, Nam Doo, Mapo-ku, Seoul (KR); Yoo, Jae Kuk, Suh-ku, Taejeon City (KR)
(74) Representative: Koepe, Gerd L., Dipl.-Chem.
(86) International application number: IB9700623
(87) International publication number: WO98053810

(56) References cited:
- WO-A-93/00928
- WO-A-94/12171
- WO-A-94/12198
- US-A- 5 157 049

## Description

The present invention relates generally to the field of pharmaceuticals, and particularly to the various pharmaceutical compositions which are used in the preparation and administration of taxol and its derivatives.

### 2. Description of the Prior Art

Taxol (paclitaxel) is one of a class of drugs called taxanes. These types of drugs promote polymerization of tubulin and stabilize the structure of intracellular microtubules. This process has the effect of inhibiting the normal dynamic reorganization of the microtubules that is necessary for interphase and mitotic functions. Taxol may also potentiate the cytotoxic effects of radiation.

In December of 1992, the Food and Drug Administration (FDA) approved the natural form of taxol for treatment of metastatic ovarian cancer after failure of first-line or subsequent chemotherapy, and the use of taxol for the treatment of metastatic breast cancer received marketing approval in April of 1994. Although taxol has shown to have effects of inhibiting the progression of cancer in some patients, it has proven to be somewhat difficult to prepare and administer. Usually applied to patients intravenously, taxol is diluted in a suitable parenteral fluid since it is available only in a somewhat viscous solution. Its insolubility in water makes administration of the drug somewhat difficult.

Therefore, a number of different compositions have been proposed to be used as solvents for preparing taxol for injection. For Example, U.S. Patent No. 5,478,860 discloses a manufacturing process mixing taxol with olive or sunflowerseed oil and polyethyleneglycol (PEG) into a microemulsion. In addition, European Patent No. 639,577 discloses a process of manufacturing the derivatives of taxol having enhanced solubility in water by way of using phosphooxymethyl (POM) or methyltheomethyl (MTM). The PCT International Patent Publication, WO-9318757, discloses a process which solves the solubility problem and enhances the pharmaceutical stability through the development of liposome-encapsulated taxol as a drug delivery system (DDS).

The document WO 94/12198 describes injectable solutions of taxol having an improved stability under a pH value of less than 8.1. The pH value is adjusted by the addition of citric acid. The composition additionally contains in preferred embodiments an emulsifier and ethanol.

The document WO 94/12171 is directed to injectable taxol compositions which additionally contain a surface-active agent and an additive selected from glucose, glycerol and ethanol.

The document WO 93/00928 relates to injectable solutions containing taxol or its derivatives in a surface-active agent and ethanol.

Presently, the most common form of solvent for preparing a taxol injection solution is a mixture of dehydrated ethanol and polyethoxylated castor oil. The stability of injection solutions prepared in this manner is limited, and the use of these solutions after prolonged storage may result in the neutralization of the taxol or induced side effects to the patient due to the dissociation of the taxol in solution.

Therefore, there is a current need for a pharmaceutical composition which is effective in the preparation of a taxol injection solution which maintains the stability of taxol in solution during prolonged storage. This is done in an effort to produce an injection solution which when administered to patients reduces the side effects normally associated with a taxol injection solution prepared using previous disclosed or conventional methods.

Accordingly, the principle object of the present invention is to provide a pharmaceutical composition which is used for the preparation of a taxol injection solution.

Another object of the present invention is to provide a pharmaceutical composition which significantly reduces the side effects normally associated with the use of conventional taxol injection solutions.

The present invention discloses a pharmaceutical composition which is used for the preparation of a taxol injection solution which is effective in clinical utilization as a cancer-inhibiting drug. The composition comprises taxol, dehydrated ethanol, polyethoxylated castor oil, calcium disodium edetate, and tromethamine. Because conventional compositions used as injection solutions are often primarily comprised of dehydrated ethanol and polyethoxylated castor oil, the components which distinguish the present invention from the prior art are calcium disodium edetate and tromethamine. Calcium disodium edetate is used as a detoxicant against poisoning from heavy metals, reduces the side effects caused by taxol, and prolongs the stability of the solution. Tromethamine (TRIS) is used as an alkalizer to alleviate breathing difficulties and is also used as a buffering agent for blood plasma. The invention also comprises the use of such compositions for preparing an cancer treatment medicament.

These together with other objects of the invention are explained clearly in the claims annexed to and forming a part of this disclosure. For a better understanding of the invention, its operating advantages and the specific objects attained by its use, reference should be made to the descriptive matter in which there are disclosed preferred embodiments of the invention.

The present invention is a pharmaceutical composition which is used for the preparation of a taxol injection solution which is effective in clinical utilization as a cancer-inhibiting drug. The addition of calcium disodium edetate and tromethamine to conventional injections solutions enhances the stability of the solution, and reduces the side effects normally associated with administration of taxol. The composition comprises taxol, dehydrated ethanol, polyethoxylated castor oil, tromethamine, and calcium disodium edetate, preferably in specified ranges. The following are some examples of preparation methods of the enhanced injection solution.

### Example 1

30 mg of tromethamine is added to 10 ml of dehydrated ethanol under the application of heat; the resulting solution is agitated for approximately 5 minutes, and then sonicated for approximately 5 minutes. The solution is brought to room temperature and 300 mg of taxol is added to the solution; the resulting solution is then agitated for approximately 5 minutes and sonicated for approximately 5 minutes. While being agitated, the resulting solution is gradually added to 25 ml of Cremophor^{R} EL. A separate solution of 10 mg calcium disodium edetate dissolved in 50 ml of 100% unhydrous ethanol is prepared and agitated for 5 minutes. This solution containing calcium disodium edetate is added to the previous solution until a 50 ml volume is achieved. The resulting solution is agitated for 10 minutes before the final solution is obtained.

### Example 2

60 mg of tromethamine is added to 10 ml of dehydrated ethanol under the application of heat; the resulting solution is agitated for approximately 5 minutes, and then sonicated for approximately 5 minutes. The solution is brought to room temperature and 300 mg of taxol is added to the solution; the resulting solution is then agitated for approximately 5 minutes and sonicated for approximately 5 minutes. While being agitated, the resulting solution is gradually added to 25 ml of Cremophor^{R} EL. Hereinafter, this solution will be referred to as solution A. A separate solution, hereinafter referred to as solution B, comprised of 5 mg calcium disodium edetate dissolved in 100% unhydrous ethanol, is prepared and agitated for 5 minutes. Solution B has a volume which would correspond to a total volume of 50 ml when solution B and solution A are mixed. The resulting mixture of solution A and B is agitated for 10 minutes before the final solution is obtained.

### Example 3

20 mg of tromethamine is added to 10 ml of dehydrated ethanol under the application of heat; the resulting solution is agitated for approximately 5 minutes, and then sonicated for approximately 5 minutes. The solution is brought to room temperature and 300 mg of taxol is added to the solution; the resulting solution is then agitated for approximately 5 minutes and sonicated for approximately 5 minutes. While being agitated, the resulting solution is gradually added to 25 ml of Cremophor^{R} EL. Hereinafter, this solution will be referred to as solution A. A separate solution, hereinafter referred to as solution B, comprised of 5 mg calcium disodium edetate dissolved in 100% unhydrous ethanol, is prepared and agitated for 5 minutes. Solution B has a volume which would correspond to a total volume of 50 ml when solution B and solution A are mixed. The resulting mixture of solution A and B is agitated for 10 minutes before the final solution is obtained.

The aforementioned examples teach specific amounts of each component which are used to produce different injection solutions. The present invention, however, comprises the use of tromethamine and calcium disodium edetate in injection solutions which have been previously used.

## Claims

1. A pharmaceutical composition used for the preparation of taxol as an injection solution comprising;
a) taxol;
b) dehydrated ethanol;
c) polyethoxylated castor oil;
d) tromethamine; and
e) calcium disodium edetate.

2. A pharmaceutical composition according to claim 1, wherein the concentration of said tromethamine in the solution is between 0.4 and 1.2 mg per ml of solution, and the concentration of said calcium disodium edetate in solution is between 0.1 and 0.2 mg per ml of solution.

3. A pharmaceutical composition according to claim 1, wherein the concentration of said taxol in solution is 6 mg per ml of solution, the concentration of said tromethamine is 0.6 mg per ml of solution, the concentration of said calcium disodium edetate in the solution is 0.1 mg per ml of solution, wherein said polyethoxylated castor oil comprises 50% of the total volume of said solution.

4. Use of the pharmaceutical composition according to any of the claims 1 to 3 for preparing a medicament for the treatment of cancer.

## Patentansprüche

1. Pharmazeutische Zusammensetzung, die verwendet wird für die Herstellung von Taxol als Injektionslösung, umfassend:
(a) Taxol;
(b) wasserfreies Ethanol;
(c) polyethoxyliertes Rizinusöl
(d) Tromethamin; und
(e) Calciumdinatriumedetat.

2. Pharmazeutische Zusammensetzung nach Anspruch 1, worin die Konzentration des Tromethamins in der Lösung zwischen 0,4 und 1,2 mg pro ml Lösung ist, und die Konzentration des Calciumdinatriumedetats in der Lösung zwischen 0,1 und 0,2 mg pro ml Lösung ist.

3. Pharmazeutische Zusammensetzung nach Anspruch 1, worin die Konzentration des Taxols in der Lösung 6 mg pro ml Lösung ist, die Konzentration des Tromethamins 0,6 mg pro ml Lösung ist, die Konzentration des Calciumdinatriumedetats in der Lösung 0,1 mg pro ml Lösung ist, worin das polyethoxylierte Rizinusöl 50 Prozent des Gesamtvolumens der Lösung umfasst.

4. Verwendung der pharmazeutischen Zusammensetzung nach einem der Ansprüche 1 bis 3 zur Herstellung eines Medikaments zur Behandlung von Krebs.

## Revendications

1. Composition pharmaceutique utilisés pour la préparation du taxol en tant que solution injectable, comprenant :
a) du taxol ;
b) de l'éthanol déshydraté ;
c) de l'huile de ricin polyéthoxylée ;
d) de la trométhamine ;
e) de l'édétate calcique et dissodique.

2. Composition pharmaceutique selon la revendication 1, dans laquelle la concentration de ladite trométhamine dans la solution est comprise entre 0,4 et 1,2 mg par ml de solution et la concentration d'édétate calcique et dissodique dans la solution est comprise entre 0,1 et 0,2 mg par ml de solution.

3. Composition pharmaceutique selon la revendication 1, dans laquelle la concentration dudit taxol dans la solution est de 6 mg par ml de solution, la concentration de ladite trométhamine est de 0,6 mg par ml de solution, la concentration d'édétate calcique et dissodique dans la solution est de 0,1 mg par ml de solution, dans laquelle ladite huile de ricin polyéthoxylée comprend 50% du volume total de ladite solution.

4. Utilisation de la composition pharmaceutique selon l'une des revendications 1 à 3 pour préparer un médicament pour le traitement du cancer.
